# EUROPEAN PATENT APPLICATION

(11) **EP 3 696 818 A1**
(43) Date of publication of application: **19.08.2020**
(21) Application number: 19157494.6
(22) Date of filing: 15.02.2019
(51) Int. Cl.: G16H 15/00, G16H 30/20, G16H 30/40

(54) **AUTOMATIC KEY IMAGE GENERATION FOR RADIOLOGY REPORTS**

(71) Applicant: Siemens Healthcare GmbH, 91052 Erlangen (DE)
(72) Inventor: SCHWEMMER, Chris, 91301 Forchheim (DE); SCHÖBINGER, Max, 96114 Hirschaid (DE); GRIMMER, Rainer, 91056 Erlangen (DE); HARTUNG; Ulrich, 91094 Langensendelbach (DE); SHARMA, Puneet, 08550 Princeton Junction, NJ (DE)

(57) **Abstract**

The invention provides a method and a system for generating a medical report based on medical imaging data. The method comprises at least the steps of:
a) providing (S10) medical imaging data;
b) providing (S20) a preliminary medical report, the preliminary medical report being at least partially based on the provided medical imaging data;
c) automatically generating (S30) a list of clinical findings within the provided preliminary medical report;
d) identifying (S40), for each clinical finding on the generated list, a corresponding anatomical location;
e) providing (S50), using the provided medical imaging data (1), at least one key image corresponding to at least one of the identified corresponding anatomical locations;
f) generating (S60) a final medical report based on the preliminary medical report and the provided key images for the identified corresponding anatomical locations.

## Description

Radiology, in particular the science of studying and interpreting medical imaging data such as CT images, MRI images, echo images and so on, is usually a service by radiologists to clinicians. The means of communication between radiologist and clinician is the so-called radiology report.

Typically, these radiology reports are pure text, making it difficult for the clinician to understand or access certain findings. Hence, the ideal report is enriched by images and/or schematic drawings that depict the identified findings. However, manually adding images to reports is time-consuming and does hardly happen in clinical routine. In addition, clinicians often do not have access to the Picture Archiving and Communication System, PACS, to look into the actual imaging data, or image evidence, themselves, or lack time or radiologic reading knowledge to do so.

If performed at all nowadays, the adding of illustrative images to the relevant findings of a medical report such as a radiology report are added manually. However, the additional time required for these images is typically not remunerated by any health insurance. Also, the radiologist's reporting workflow is interrupted every time they would like to add a snapshot or image. Additionally, radiologists are not typically media experts and therefore spend significant amounts of time selecting, cropping, transferring and pasting images, which is not an efficient economic use of their valuable time.

In the prior art, natural language processing (NLP) technology is known which can be used to analyze a text and to extract information from it, for example as described in US patent application 15/957,143.

It is an object of the present invention to provide a method and a system for simply, and with little effort, provide high-value medical reports, in particular to enrich medical reports with specifically selected highly relevant images.

These objects are solved by the subject-matter of the independent claims.

According to a first aspect, a computer-implemented method for generating a medical report based on medical imaging data as provided, comprising the steps of:
a) providing medical imaging data;
b) providing a preliminary medical report, the preliminary medical report being at least partially based on the provided medical imaging data;
c) automatically generating a list of clinical findings within the provided preliminary medical report;
d) identifying, for each clinical finding on the generated list, a corresponding anatomical location;
e) providing, using the provided medical imaging data, at least one key image corresponding to at least one of the identified corresponding anatomical locations;
f) generating a final medical report based on the preliminary medical report and the provided key images for the identified corresponding anatomical locations.

Medical imaging data may comprise, or consist of, medical images produced by any known, or even currently unknown, medical imaging technique (or: modality) such as computed tomography, CT, magnetic resistance imaging, MRI, X-ray imaging, ultrasound imaging and/or the like.

The preliminary medical report being at least partially based on a medical imaging data shall be understood to mean that the preliminary medical report may be a diagnosis, a summary, a written conclusion or the like generated by a user, in particular a radiologist, when analyzing the provided medical imaging data, although of course the user will also apply their professional knowledge, training and expertise as well.

Clinical findings within the provided preliminary medical report may comprise symptoms, medical diagnoses, mentions of anomalies and/or the like.

A corresponding anatomical location may be understood to be an anatomical location corresponding to an anatomical part within the medical imaging data that is mentioned in the item on the generated list, which is an anatomical portion that is affected by the item, in which the item has been described to be present according to the preliminary medical report and/or the like.

An image corresponding to at least one of the identified corresponding anatomical locations may be understood to be an image, which shows said anatomical location, for example in its healthy state, its most common state, in a diseased or afflicted state or in the state according to the medical imaging date, or any combination thereof. Of course, multiple images may be provided corresponding to each of the at least one of the identified corresponding anatomical locations.

For example, if an anomaly has been found within a certain anatomical location according to the generated list of clinical findings, in step e) both an actual picture of said anatomical location from, or based on, the medical imaging data may be provided along with a corresponding (that is, preferably taken from the same angle, with the same zoom factor and/or the like) from an external database, which shows the anatomical location without said anomaly.

Providing the at least one images may also comprise adding visual indications automatically, such as errors to guide the eye towards the mentioned anomaly, in case of multiple images preferably showing to the same spot within the images, indicating that the anomaly is present in one image and is not present in the other image or the like. Instead of an error, the spot of the anomaly may be also indicated in other visual ways, for example by circling it, by putting spotlight effect on it (that it, by fading out or darkening the area of the respective image side the spot of interest containing the anomaly) and so on.

It shall be understood that the decisions to be made in steps c) through e) are preferably made based on any suitable algorithm. For example, a database could be provided which labels certain key words within the preliminary medical report as clinical findings and/or which links said clinical findings then individually, or in groups, to corresponding anatomical locations and/or which links the anatomical locations and/or the clinical findings with parameters of the key image to be provided.

For example, the key phrase "obstruction in artery x" may be labeled as a clinical finding and as corresponding to the "artery x" as its anatomical location. Moreover, the database may indicate that the clinical finding of obstruction of an artery should lead to a key image being provided, which shows a longitudinal section through said artery x, ideally taken from medical imaging data of a specific medical exam that best illustrates artery obstructions if imaging data of that exam are present within the medical imaging data provided in step a) .

It will be apparent that a large number of such rules can be divided and set up, the specific implementation of which will depend on the type of medical imaging data, the images available, the clinical findings to be deemed to be most relevant, the functionalities of an image managing system (such as a PACS) regarding the generation of three-dimensional images and new cross-sections, and so on.

It will also be understood that at least one of the steps c) through e), preferably all of these steps, may be performed using a trained artificial neural network.

The term "final medical report" is used herein to differentiate said final medical report from the preliminary medical report, but should not be understood to mean that this "final medical report" cannot be altered in any way further after the described steps. After the steps as described herein, the final medical report comprises parts of the preliminary medical report, in particular all of the preliminary medical report, enriched with at least one key image, preferably enriched with at least one key image for each item on the generated list of clinical findings within the provided preliminary medical report.

The term "key image" is used herein to designate the images that are provided in step e) and which are used in the final medical report, wherein the phrase "key image" is chosen to indicate that these images are intended to be important illustrations for the most relevant or most important clinical findings so as to support the work of the clinicians, which have to act on the final medical report (such as a radiology report).

With the method as described above, the radiologist does not have to necessarily take care of any image manipulation, selection, pasting and so on. Instead, the radiologist can focus simply on providing the preliminary medical report; nevertheless, the generated final medical report will comprise at least one key image, preferably at least one key image for each of the clinical findings within the provided preliminary medical report, which will greatly enhance the scientific and medical value of the final medical report for later users such as clinicians.

In some advantageous embodiments, a further step comprises providing the key image and/or the final medical report, wherein providing the key image and/or the final medical report comprises displaying, transmitting and/or storing the key image and/or the final medical report.

In some advantageous embodiments, in step e) at least one of the key images is provided by retrieving it from the provided medical imaging data. In other words, an image already comprised in the medical imaging data provided in step a) can be selected and retrieved to provide it in step e) as one of the at least one key images. This makes it immediately comprehensible for a clinician based on which of the medical images present in the provided medical imaging data the clinical findings is based on, or at least, to which anatomical location the clinical finding is related.

In some advantageous embodiments, in step e) at least one of the key images is provided by generating it based on the provided medical imaging data. For example, medical imaging data are often provided in distinct slices, each taken an almost negligibly different time than the other. Three-dimensional images may be constructed by assembling a plurality of such slices and by interpolating the spaces in between the slices, by producing a voxel structure based on the plurality of slices and/or the like. New cross-sections, even particular once to the original slices, may be generated either based on the original slices or based on the generated three-dimensional structure. In this way, even cross-sections that are not part of the original medical imaging data but that may best illustrate a particular clinical finding or the like can be automatically generated and provided as the key image.

In some advantageous embodiments, the composing of the preliminary medical report is performed at least in part by typing. In other words, the method may comprise a user, in particular a radiologist, type at least part of, or all of, the preliminary medical report. Preferably, at least some of the steps of the method may then be performed simultaneously with the typing of the preliminary medical report. In this way, the user may immediately review the results of the method and may even, in this way, be assisted in preparing the preliminary medical report.

For example, when a specific key image is automatically inserted into the preliminary medical report as the user is typing it, the user may become aware of another issue or may even revise their diagnosis, for example delete the corresponding type parts of the preliminary medical report and replace them with others. For example, when the user has identified a very specific condition, but has typed only a more abstract description, the user may be immediately provided with a key image that is evidently not very meaningful.

The user may then, for example, backtrack and replace the original phrasing with a more pertinent one. As a result, the provided image may be more relevant and the user may then continue with typing the preliminary medical report.

In some advantageous embodiments, the composing of the preliminary medical report is performed at least apart by dictating and by automatic speech-to-text conversion of the dictated text. Medical practitioners, in particular radiologists, often dictate their preliminary medical reports while reviewing the medical imaging data. It is therefore advantageous when, as described above in the case of typing, the user is provided with key images "on the fly", that is, concurrently with the composing of the report. In this way, the present method may also be designated as a method for assisting in preparing a medical diagnosis.

Advantageously, whenever a clinical finding is detected in the preliminary medical report as it is being composed (typed and/or dictated), steps d) and e) may be performed and a key image may be provided and displayed to the user composing the preliminary medical report, either automatically inserted into the preliminary medical report when it is visible as text, or on a computer screen or monitor. This provides immediate visual corroboration and/or feedback to the user of whether the most recent clinical finding comprised in the preliminary medical report as it is being composed is correct.

Moreover, the method in this sense also provides a tool for improved diagnosis. For example, when the original medical imaging data do not show a specific cross-section that would be ideal for determining whether a certain condition is present in a certain anatomical location, the user may tentatively enter a corresponding clinical finding such that, according to the method, a corresponding key image is provided, for example, according to predefined rules and/or a trained artificial neural network, exactly the image within the ideal cross-section. The user can then decide whether the tentative diagnosis was correct and continue, or whether the tentative diagnosis was incorrect and backtrack.

The speech-to-text conversion can be provided according to any of the known techniques in the art.

In some advantageous embodiments, during the composing of the preliminary medical report accessing information is collected automatically. Accessing information is understood to be information indicating images of the received medical imaging data being accessed, or viewed, by a user performing the composing of the preliminary medical report.

In other words, it may be registered, which images of the received medical imaging data the user has viewed, or at least were displayed at a screen intended to be viewed by the user, during the time that a certain text passage was typed or dictated by the user. This means in particular that the accessing information may indicate, which images are or were accessed, or viewed, by the user during which periods of the composing of the preliminary medical report. Presumably, this means that said image was at least part of the cause why the user has entered that specific text passage into the preliminary medical report.

Accordingly, this image is usually a good candidate for being provided as the key image for any clinical findings, which may be present in said text passage. Advantageously, in step d) the identifying of the corresponding anatomical location for each item on the generated list is performed at least partially based on the collected accessing information. Similarly, also the providing in step e) of the key image may be based at least partially on the collected accessing information.

For example, when in a specific sentence entered into the preliminary medical report a clinical finding has been identified, the accessing information can be accessed by a computing device performing the method to find out which image of the received medical imaging data was viewed by the user when said sentence was entered.

A label of said image indicating an anatomic location may then be used to identify in step d) the corresponding anatomical location, and/or the image itself may be used as the corresponding key image, or at least may be used to generate (possibly in combination with additional images) the corresponding key image.

In some advantageous embodiments, step c) and/or step d) is (or: are) at least partially performed during the composing of the preliminary medical report. As described above, the clinical findings within the provided preliminary medical report may be identified "on the fly" as they are being entered into the preliminary medical report. In the same way, for each clinical finding, as it is being determined within the provided preliminary medical report in order to be added to the automatically generated list of clinical findings, immediately the corresponding anatomical location may be identified. In this way, computational resources present during the composing of the preliminary medical report, which may otherwise be unused, can be employed to save time and to allow the final medical report being ready as soon as possible.

In some advantageous embodiments, step e) is at least partially performed during the composing of the preliminary medical report. As has been described already to some degree in the foregoing, when the key images are provided "on the fly", they may be immediately displayed to the user composing the preliminary medical report, thus supporting the diagnosis by the user and/or allowing the user to confirm or reject said image as relevant key image.

In some advantageous embodiments, for at least one of the identified corresponding anatomical locations, in step e) an image is provided by automatically retrieving or generating a schematic drawing. Such a schematic drawing may help a recipient of the final medical report, such as a clinician, to better understand the anatomical details about a specific clinical finding.

Preferably, said generated schematic drawing is provided together with an actual image of the same anatomical location based on, or retrieved from, the provided medical imaging data so that the recipient of the final medical report can immediately associate the actually shown anatomical locations with the once in the generated schematic drawing. Generating the schematic drawing may be performed by a trained algorithm. Retrieving the schematic drawings may be performed by accessing a database comprising a plurality of schematic drawings for a variety of anatomical locations, preferably dependent on clinical findings as well. In that way, for a clinical finding A associated with anatomical location X, a different schematic drawing may be generated or retrieved than for a clinical finding B in connection with an anatomical location X. This helps to always provide the most relevant key image for understanding and/or corroborating a clinical finding.

In some advantageous embodiments, for at least one of the identified corresponding anatomical locations, in step e) a key image is provided by retrieving it from an image repository separate from the received medical imaging data. Said image repository may, for example, be a PACS. Preferably, the key image retrieved in this way is an image from another medical exam, that is, an image of an actual human organ provided by an actual medical exam. For example, when the clinical finding is a diseased organ X, an at least one of the provided key images for that clinical finding may be provided from previous exams done on previous patients with the same condition.

In some advantageous embodiments, the key image and/or the final medical report are used in a decision support system. In general, a decision support system can prepare and display medical data corresponding to a patient, in order to enable an operator or physician to take a well-founded decision about next steps in the diagnosis or therapy of the patient. In particular, the key image can be generated by the decision support system in preparing and/or displaying the medical data corresponding to the patient, in order to provide the key image as an additional source of information to the operator or physician.

According to a second aspect, the invention provides a system for generating a medical report based on medical imaging, comprising:
an imaging data providing module configured to provide medical imaging data;
a report providing module configured to provide a preliminary medical report, the preliminary medical report being at least partially based on the medical imaging data;
a scanning module configured to automatically generate a list of clinical findings within the provided preliminary medical report;
an identifying module configured to identify, for each clinical finding on the generated list, a corresponding anatomical location;
an image providing module configured to provide, using the provided medical imaging data, a key image for at least one of the identified corresponding anatomical locations;
a report generating module configured to generate a final medical report based on the preliminary medical report and the provided key images for the identified corresponding anatomical locations.

The modules may be realized in hardware, such as a circuit or a printed circuit board and/or comprising transistors, logic gates and other circuitry. Additionally, the modules may be at least partially realized in terms of software. Accordingly, the modules may comprise, or be operatively coupled to, a processor and a memory storing a software or a firmware that is executed by the processor to perform the functions of the modules. Signals may be received by an input interface of the modules and signals that the processor of the modules creates may be outputted by an output interface of the modules. The modules may be implemented, at least partially, as a microcontroller, an ASIC, an FPGA and so on.

Any or all of the modules described in the foregoing may be implemented by a computing device or may be connected to a computing device.

The computing device may be realized as any device, or any means, for computing, in particular for executing a software, an app, or an algorithm. For example, the computing device may comprise a central processing unit (CPU) and a memory operatively connected to the CPU. The computing device may also comprise an array of CPUs, an array of graphical processing units (GPUs), at least one application-specific integrated circuit (ASIC), at least one field-programmable gate array, or any combination of the foregoing.

Some, or even all, modules of the system may be implemented by a cloud computing platform.
Further advantageous embodiments, variants, options and modifications are apparent from the dependent claims as well as from the description in combination with the figures.

In systems based on cloud computing technology, a large number of devices is connected to a cloud computing system via the Internet. The devices may be located in a remote facility connected to the cloud computing system. For example, the devices can comprise, or consist of, equipments, sensors, actuators, robots, and/or machinery in an industrial set-up(s). The devices can be medical devices and equipments in a healthcare unit.

The cloud computing system may comprise a plurality of servers or processors (also known as 'cloud infrastructure'), which are geographical distributed, connected with each other via a network. A dedicated platform (hereinafter referred to as 'cloud computing platform') is installed on the servers/processors for providing above functionality as a service (hereinafter referred to as 'cloud service'). The cloud computing platform may comprise a plurality of software programs executed on one or more servers or processors of the cloud computing system to enable delivery of the requested service to the devices and its users.

In some advantageous embodiments, the system further comprises a dictation module configured to receive natural speech as input and to provide at least a part of the preliminary medical report by transcribing the received natural speech. In other words, a user such as a radiologist may compose the preliminary medical report by speaking naturally into a microphone.

According to a third aspect of the invention, a computer program product is provided comprising executable program code configured to, when executed by a computing device, perform the method according to an embodiment of the first aspect of the invention.

According to a fourth aspect of the invention, a non-transitory computer-readable data storage medium is provided, which comprises executable program code configured to, when executed by a computing device, performed a method according to an embodiment of the first aspect of the invention.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like.

According to a fifth aspect, the invention provides a data stream comprising, or configured to generate, executable program code configured to, when executed by a computing device, perform the method according to an embodiment of the first aspect of the present invention.

The invention will be explained in yet greater detail with reference to exemplary embodiments depicted in the drawings as appended.

The accompanying drawings are included to provide a further understanding of the present invention are incorporated in and constitute a part of the specification. The drawings illustrate the embodiments of the present invention and together with the description serve to illustrate the principles of the invention. Other embodiments of the present invention and many of the intended advantages of the present invention will be readily appreciated as they become better understood by reference to the following detailed description. Like reference numerals designate corresponding similar parts.

The numbering of method steps is intended to facilitate understanding and should not be construed, unless explicitly stated otherwise, to mean that the designated steps have to be performed according to the numbering of their reference signs. In particular, several or even all of the method steps may be performed simultaneously, in an overlapping way or sequentially.
- Fig. 1: shows a schematic flow diagram illustrating a method according to an embodiment of the first aspect of the present invention;
- Fig. 2: shows a schematic block diagram illustrating a system according to an embodiment of the second aspect of the present invention;
- Fig. 3: shows a schematic block diagram of a computer program product according to an embodiment of the third aspect of the present invention; and Fig. 4 shows a schematic block diagram of a data storage medium according to an embodiment of the fourth aspect of the present invention.

Although specific embodiments have been illustrated and described herein, it will be appreciated by those of ordinary skill in the art that the variety of alternate and/or equivalent implementations may be substituted for the specific embodiments shown and described without departing from the scope of the present invention. Generally, this application is intended to cover any adaptations or variations of the specific embodiments discussed herein.

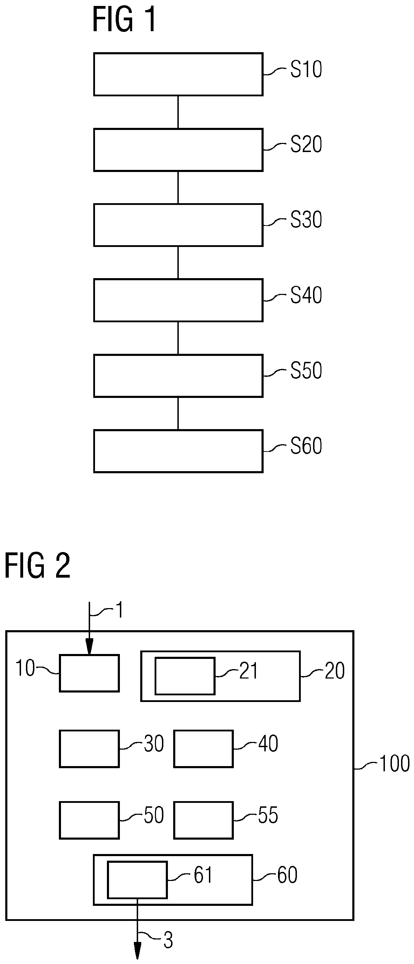

Fig. 1 shows a schematic flow diagram illustrating a computer-implemented method for generating a medical report based on medical imaging data according to an embodiment of the first aspect of the present invention.

In a step S10, medical imaging data are provided. The medical imaging data may be imaging data from one or more of the following exams:
- a computed tomography (CT) exam;
- magnetic resonance imaging (MRI) exam;
- nuclear imaging exam;
- ultrasound imaging exam;
- X-ray scan exam;
- and/or the like.

The medical imaging data may be provided S10 directly by a medical imaging device and/or by a picture archiving and communication system (PACS). Medical imaging data may be provided of a local connection within a tenant's site such as over an Intranet network of a hospital or medical research institute. The medical imaging data may also be provided S10 at least partially over a wide area network connection such as the Internet. Some or all of the steps of this method may be performed in a cloud computing system.

In a step S20, a preliminary medical report is provided, the preliminary medical report being at least partially based on the provided medical imaging data, in particular in that a radiologist has composed the preliminary medical report based on, and possible during viewing, the medical imaging data.

In some variants, the preliminary medical report may be provided as a finished document, which may then be further processed by the present method. In many embodiments and variants, however, the preliminary medical report is being composed during the runtime of at least part of the method so that one or more method steps can be performed "on the fly", i.e. during the composing of the preliminary medical report by a user such as a radiologist.

For example, the user may type the preliminary medical report or may use a dictation system in which natural speech of the user is captured by a microphone and is rendered into text by a speech-to-text system or a dictation module run on a computing device, which transcribes the captured natural speech into written text.

In a step S30, a list of clinical findings within the provided preliminary medical report is automatically generated. Generating S30 the list may comprise collecting clinical findings in at least one field of a structured preliminary medical report and/or scanning the preliminary medical report in order to determine clinical findings mentioned therein. In case that the preliminary medical report is a structured report, in which, for example, certain fields relating to clinical findings are filled in, for instance by inserting certain code numbers that indicate certain clinical findings, the clinical findings can be directly read out from the preliminary medical report by an algorithm designed, or trained, for this task.

In case of a free-text report, natural language processing (NLP) technology can be used to analyze the preliminary medical report and to extract the clinical findings from within for generating the list of the clinical findings. Such techniques are, for example, described in US patent application 15/957,143.

As mentioned before, step S30 may already be performed at least partially during the composing of the preliminary medical report, for example during typing and/or during dictation of the preliminary medical report by a user. In this way, the time spent by the user in composing the report is not wasted, but is used to perform computational steps, which in some instances may require considerable time.

In a step S40, for each item on the generated list (that is, for each clinical finding on the generated list of clinical findings), a corresponding anatomical location is identified. As has been described in the foregoing, this may comprise utilizing a database, which links certain key phrases, key words, word combinations and so on of a free text, and/or certain entries in certain fields of a structured preliminary medical report, with such anatomical locations.

In particular, clinical findings formulated in natural language may themselves already comprise the mention of one or more anatomical locations such as a specific organ, part of an organ, blood vessel, bone or bone structure, and/or the like. In these cases, the corresponding anatomical location may be identified simply by reading out the anatomical location mentioned in said clinical finding.

In a step S50, using the provided medical imaging data, at least one key image corresponding to at least one of the identified corresponding anatomical locations is provided. The providing of the key image may comprise retrieving the key image, in particular from the provided medical imaging data. Providing of the at least one key image may also comprise, or consist of, generating a key image, preferably based on the provided medical imaging data.

For example, a certain clinical finding may in the database be associated with a particular view (for example, a specific cross-section in a specific plane through a specific organ), which may not be originally present in the provided medical imaging data. In that case, providing S50 at least one key image may comprise generating, using interpolation and/or extrapolation between one or a plurality of images within the provided medical imaging data, said particularly advantageous view.

For example, for certain blood vessels a three-dimensional view may be the most appropriate to determine, for example, an anomalous formation of said blood vessel. In that case, a three-dimensional image may be automatically generated from the medical imaging data provided in step S10 even if therein originally only two-dimensional images are present.

Providing S50 the at least one key image may also comprise automatically retrieving or generating a schematic drawing related to, or depicting, the identified corresponding anatomical location. It is especially preferred that for one clinical finding both at least one image retrieved from, or based on, the provided medical imaging data is provided in step S50, together with an automatically retrieved or generated schematic drawing showing the same anatomical location, most preferably viewed from the same perspective as the "real" image (i.e. the image from an actual exam or generated from data of one or more actual exams).

In that way, a recipient of the final medical report is immediately presented with not only the actual imaging result in form of the retrieved and/or generated images based on the provided medical imaging data, but also with a schematic drawing illustrating what the recipient is supposed to perceive, which may greatly enhance the intelligibility of the provided key images and thus also of the final medical report itself.

It is preferred that for every clinical finding on the generated list of clinical findings at least one key image is provided. More preferably, for each clinical finding of the generated list of clinical findings both a key image based on the provided medical imaging data is provided as well as a schematic drawing.

In some embodiments, the schematic drawing may be augmented by, or replaced with, a standardized image trying the same an anatomical location, more preferably from the same viewpoint, and depicting a healthy and/or a diseased patient. Thus, the provided key images for at least one, preferably for all clinical findings of the generated list of clinical findings, may comprise any or all of:
- an actual image for the patient for which the preliminary medical report has been composed and which is either retrieved from (e.g. a "snapshot"), or generated based on, the provided medical imaging data for that patient;
- a schematic drawing, preferably viewed from the same perspective as the actual image, showing the same anatomical location and optionally labeling some of the shown organs and/or organ structures;
- a generic image of the same anatomical location, preferably viewed from the same perspective, showing the same location in a healthy patient; and/or
- an image showing the same anatomical location, preferably viewed from the same perspective, of a diseased patient.

In particular, when all four of these images are provided, it may be especially easy for the recipient of the final medical report to understand what is shown in the medical imaging data of the patient and how it relates to a healthy and a diseased system, respectively, so as to be able to spot the similarities and/or differences to either or both.

A providing of the at least one key image in step S50 can be performed simply based on the anatomical location. However, also additional information contained in the identified clinical finding may be used to influence the selection of the at least one key image being provided.

For example, the perspective chosen may depend on the clinical finding that has been entered into the preliminary medical report for the anatomical location, and/or even a type of key image being provided may depend on said information. For example, key images of one of a plurality of modalities may be selected based on the information contained within the clinical finding separate from the information about the anatomical location. Also the type of key image being provided in step S50 (for example: parallel or radial range, curved reformation, volume rendering and/or the like) can be adapted to the type of clinical finding extracted from the preliminary medical report.

In step S50, apart from the at least one key image being provided, also additional information can be provided, which is neither present in the preliminary medical report nor in the provided medical imaging data. Such additional information may, for example, be retrieved from previous imaging studies (for example: suggested causes or therapies for said clinical finding or previous states of the same anatomical region illustrating changes in disease) or from an electronic medical record, EMR, system.

As has been mentioned before, also step S50 may be provided, at least partially or preferably completely, during the composing of the preliminary medical report in cases, in which the preliminary medical report is not provided as a finished document.

In some advantageous embodiments, a computer screen or display may be provided in the location where the user is composing the preliminary medical report and the key images (and optionally the additional information) being provided in step S50 may be displayed to the user even during the composing of the preliminary medical report.

As has been described, this would only allow to user to verify and confirm that - in their opinion - relevant and meaningful key images are provided, but may also serve as a diagnostic support tool in that the user can view the provided key images and decide whether their diagnosis is still correct given what is displayed in those key images. Since the user is in these embodiments currently composing the preliminary medical report, the user may, as a result, even alter the preliminary medical report to find a better diagnosis, that is, to enter an improved clinical finding.

In a step S60, a final medical report is generated based on the preliminary medical report and the provided key images for the identified corresponding anatomical locations. In particular, the final medical report may comprise the preliminary medical report, which is enriched by the provided key images, in particular such that the provided key images are present in the final medical report in the passages of the preliminary medical report, which mentioned the corresponding clinical finding.

Preferably, the provided key images (and optionally the additional information as described with respect to step S50 above) are inserted into the preliminary medical report right after the expression of the corresponding clinical finding. In that way, the recipient of the final medical report is presented immediately with a visualization and/or explanation of the clinical finding where it is mentioned.

In variants wherein the preliminary medical report is provided by being composed by the user "on the fly", also the provided key images (and optionally the additional information) may be inserted into the preliminary medical report as it is being composed. In this way, a computer display or a screen that is used for displaying to the user the preliminary medical report as it is being composed may also be used to display the provided key images at the same time, which has the additional advantage that the user may review the form of the final medical report while composing the preliminary medical report.

In other words, in these variants the final medical report is generated "on the fly" basically concurrently with the providing S20, i.e. composing, of the preliminary medical report. Expressed yet in another way, steps S20 through S60 are performed essentially simultaneously or concurrently or at least in a temporarily overlapping way.

The generating S60 of the final medical report may also optionally comprise scanning the text of the preliminary medical report for predetermined key phrases and replacing those predetermined keywords with pre-associated additional key images. In this way, the user (e.g. radiologist) may, during the composing of the preliminary medical report, give explicit instructions what is to be shown and how instead of having to rely completely on the automated method as described herein. Accordingly, this variant is particularly useful when the providing S20 of the preliminary medical report consists of providing the preliminary medical report as an already finished document. The additional key images may be provided in the same way as described with respect to step S50.

For example, the occurrence of the key phrase "<<spine sag VRT>>" could be automatically replaced by a volume-rendered sagittal view of the spine (e.g. of a generic spine, a healthy spine, a diseased spine and/or the spine of the patient from which the provided medical imaging data stem).

It is evident that the described method is a significant improvement in both efficiency and quality of diagnosis as well as in preparation of medical reports for recipients which may not have a deep understanding of radiology.

Fig. 2 shows a system 100 for generating a medical report based on medical imaging data according to an embodiment of the second aspect of the present invention. The system 100 of Fig. 2 may be configured to perform the method according to an embodiment of the first aspect of the present invention, in particular the embodiment as described with respect to Fig. 1. Therefore, any advantageous variations, modifications and options as described for the method according to the first aspect of the invention, in particular the method of Fig. 1, may equally be applied to the system according to the second aspect, in particular of Fig. 2, and vice versa. However, the method may also be performed on different systems than the systems according to the second aspect of the present invention.

The system 100 comprises an imaging data providing module 10 configured to provide medical imaging data 1, for example as has been described with respect to step S10 of the method of Fig. 1. In particular, the imaging data providing module 10 may be an interface for connecting to a local and/or wide area network, in particular to a Picture Archiving and Communication System (PACS).

The system 100 further comprises a report providing module 20 configured to provide a preliminary medical report, the preliminary medical report being at least partially based on the medical imaging data 1 that is provided by the imaging data providing module 10. The report providing module 20 may be configured to provide the preliminary medical report as described with respect to step S20 of the method of Fig. 1.

In particular, the report providing module 20 may be realized as a local network and/or wide area network interface configured to receive the preliminary medical report as a finished document. Additionally or alternatively, the report providing module 20 may be configured to allow a user to compose the preliminary medical report, either by typing and/or by dictating.

The report providing module 20 may therefore in some embodiments comprise a dictation module 21 configured to receive natural speech as input and to provide at least a part of the preliminary medical report by transcribing the received natural speech into text. It will be understood that the system 100 may, depending on the realization of the report providing module 20, comprise a user interface (a keyboard, a touch-screen, a computer mouse, a microphone and/or the like), a computer screen or monitor for displaying text and/or images, an acoustic output interface (for example for replaying dictated text) and/or the like. It will be understood that such components may be realized by any generated computing system adapted for the purposes and functionality of the report providing module 20.

The system 100 further comprises a scanning module 30 configured to automatically generate a list of clinical findings within the provided preliminary medical report, in particular as has been described with respect to step S30 of the method according to Fig. 1.

An identifying module 40 of the system 100 is configured to identify, for each clinical finding or a generated list of clinical findings, a corresponding anatomical location, in particular as has been described with respect to step S40 of the method according to Fig. 1.

The system 100 further comprises an image providing module 50 configured to provide, using the providing medical imaging data, at least one key image for at least one of the identified corresponding anatomical locations. The image providing module 50 may provide the at least one key image preferably as has been described with respect to step S50 of the method according to Fig. 1.

The system 100 may also comprise an information providing module 55 configured to provide additional information for at least one of the identified corresponding anatomical locations, in particular as has been described with respect to step S50 of the method according to Fig. 1.

The system 100 also comprises a report generating module 60 configured to generate a final medical report 3 based on the preliminary medical report and the provided key images for the identified corresponding anatomical locations.

The report generating module 60 may be configured to generate the final medical report 3, for example as has been described with respect to step S60 the method of Fig. 1. The report generating module 60 may comprise an output interface 61 configured to output the generated final medical report 3 from the system 100, for example to transmit the final medical report automatically to a recipient, either over a local network connection (such as an Intranet) and/or a wide area network connection (such as the Internet).

It will be understood that the system 100 may in particular comprise a computing device 101, which is configured to implement the modules 10, 20, 30, 40, 50, 55, 60 as described in the foregoing.

The modules 10, 20, 30, 40, 50, 55, 60 may also be realized in hardware, such as a circuit or a printed circuit board and/or comprising transistors, logic gates and other circuitry. Additionally, the modules 10, 20, 30, 40, 50, 55, 60 may be at least partially realized in terms of software. Accordingly, the modules 10, 20, 30, 40, 50, 55, 60 may comprise, or be operatively coupled to, a processor and a memory storing a software or a firmware that is executed by the processor to perform the functions of the modules 10, 20, 30, 40, 50, 55, 60. Signals may be received by an input interface of the modules 10, 20, 30, 40, 50, 55, 60 and signals that the processor of the modules 10, 20, 30, 40, 50, 55, 60 creates may be outputted by an output interface of the modules 10, 20, 30, 40, 50, 55, 60. The modules 10, 20, 30, 40, 50, 55, 60 may be implemented, at least partially, as a microcontroller, an ASIC, an FPGA and so on.

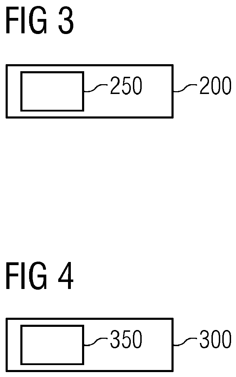

Fig. 3 shows a schematic block diagram of a computer program product 200, which comprises an executable program code 250 configured to, when executed by a computing device, perform the method according to an embodiment of the first aspect of the invention, in particular a method of Fig. 1.

Fig. 4 shows a schematic block diagram of a non-transitory, computer-readable data storage medium 300 comprising executable program code 350 configured to, when executed by a computing device, perform the method according to the first aspect of the present invention, in particular the method of Fig. 1.

In the foregoing detailed description, various features are grouped together in the examples with the purpose of streamlining the disclosure. It is to be understood that the above description is intended to be illustrative and not restrictive. It is intended to cover all alternatives, modifications and equivalence. Many other examples will be apparent to one skilled in the art upon reviewing the above specification, taking into account the various variations, modifications and options as described or suggested in the foregoing.

## Claims

1. A computer-implemented method for generating a medical report based on medical imaging data (1), comprising the steps of:
a) providing (S10) medical imaging data (1);
b) providing (S20) a preliminary medical report, the preliminary medical report being at least partially based on the provided medical imaging data (1);
c) automatically generating (S30) a list of clinical findings within the provided preliminary medical report;
d) identifying (S40), for each clinical finding on the generated list, a corresponding anatomical location;
e) providing (S50), using the provided medical imaging data (1), at least one key image corresponding to at least one of the identified corresponding anatomical locations;
f) generating (S60) a final medical report (3) based on the preliminary medical report and the provided key images for the identified corresponding anatomical locations.

2. The method of claim 1,
wherein in step e) at least one of the key images is provided by retrieving it from the provided medical imaging data (1) .

3. The method of claim 1 or claim 2,
wherein in step e) at least one of the key images is provided by generating it based on the provided medical imaging data (1).

4. The method of any of claims 1 to 3,
wherein providing (S20) the preliminary medical report comprises composing the preliminary medical report.

5. The method of claim 4,
wherein the composing of the preliminary medical report is performed at least in part by typing.

6. The method of claim 4 or claim 5,
wherein the composing of the preliminary medical report is performed at least in part by dictating and by automatic speech-to-text conversion of the dictated text.

7. The method of any of claims 4 to 6,
wherein, during the composing of the preliminary medical report, accessing information is collected automatically, the accessing information indicating images of the received medical imaging data being accessed, or viewed, by a user performing the composing;
wherein in step d) the identifying of the corresponding anatomical location for each item on the generated list is performed at least partially based on the collected accessing information.

8. The method of claim 7,
wherein the accessing information indicates which images are or were accessed, or viewed, by the user during which periods of the composing.

9. The method of any of claims 4 to 8,
wherein step c) and/or step d) is at least partially performed during the composing of the preliminary medical report.

10. The method of any of claims 4 to 9,
wherein step e) is at least partially performed during the composing of the preliminary medical report.

11. The method of any of claims 1 to 10,
wherein, for at least one of the identified corresponding anatomical locations, in step e) a key image is provided by automatically retrieving or generating a schematic drawing.

12. The method of any of claims 1 to 11,
wherein, for at least one of the identified corresponding anatomical locations, in step e) a key image is provided by retrieving it from an image repository separate from the received medical imaging data.

13. A system (100) for generating a medical report based on medical imaging data (1), comprising:
an imaging data providing module (10) configured to provide (S10) medical imaging data (1);
a report providing module (20) configured to provide (S20) a preliminary medical report, the preliminary medical report being at least partially based on the medical imaging data (1) ;
a scanning module (30) configured to automatically generate (S30) a list of clinical findings within the provided preliminary medical report;
an identifying module (40) configured to identify (S40), for each clinical finding on the generated list, a corresponding anatomical location;
an image providing module (S50) configured to provide (S50), using the provided medical imaging data (1), a key image for at least one of the identified corresponding anatomical locations; and
a report generating module (S60) configured to generate a final medical report (3) based on the preliminary medical report and the provided key images for the identified corresponding anatomical locations.

14. The system (100) of claim 13, further comprising a dictation module (55) configured to receive natural speech as input and to provide at least a part of the preliminary medical report by transcribing the received natural speech.

15. A computer program product (200) comprising executable program code (250) configured to, when executed, perform the method according to any of claims 1 to 11.
